# EUROPEAN PATENT SPECIFICATION

(11) **EP 0 249 188 B1**
(45) Date of publication and mention of the grant of the patent: **14.09.1994**
(21) Application number: 87108292.1
(22) Date of filing: 09.06.1987
(51) Int. Cl.: C12P 13/04, C12N 9/10

(54) **Process for the production of L-2-amino-4-(hydroxymethyl-phosphinyl)-butyric acid**
Verfahren zur Herstellung von L-2-Amino-4-(hydroxymethyl-phosphinyl)-Buttersäure
Procédé de production de l'acide L-2-amino-4-(hydroxyméthyl-phosphinyl)-butyrique

(30) Priority: 09.06.1986 JP 131743/86; 23.01.1987 JP 12513/87; 30.03.1987 JP 74476/87; 25.04.1987 JP 101152/87
(43) Date of publication of application: 16.12.1987
(73) Proprietor: MEIJI SEIKA KAISHA LTD., Chuo-ku Tokyo 104 (JP)
(72) Inventor: Imai, Satoshi c/o MEIJI SEIKA KAISHA LTD, Saiwai-ku Kawasaki-shi Kanagawa-ken (JP); Takane, Nobuhiko c/o MEIJI SEIKA KAISHA LTD, Saiwai-ku Kawasaki-shi Kanagawa-ken (JP); Yoshizawa, Yachiyo c/o MEIJI SEIKA KAISHA LTD, Saiwai-ku Kawasaki-shi Kanagawa-ken (JP); Saito, Toshinori c/o MEIJI SEIKA KAISHA LTD, Saiwai-ku Kawasaki-shi Kanagawa-ken (JP); Ogawa, Hiroshi c/o MEIJI SEIKA KAISHA LTD, Saiwai-ku Kawasaki-shi Kanagawa-ken (JP); Takabe, Hidehi c/o MEIJI SEIKA KAISHA LTD, Saiwai-ku Kawasaki-shi Kanagawa-ken (JP); Sato, Atsuyuki c/o MEIJI SEIKA KAISHA LTD, Saiwai-ku Kawasaki-shi Kanagawa-ken (JP); Fukatsu, Shunzo c/o MEIJI SEIKA KAISHA LTD, Saiwai-ku Kawasaki-shi Kanagawa-ken (JP); Okada, Akira c/o MEIJI SEIKA KAISHA LTD, Saiwai-ku Kawasaki-shi Kanagawa-ken (JP); Murakami, Takeshi c/o MEIJI SEIKA KAISHA LTD, Kohhoku-ku Yokohama-shi Kanagawa-ken (JP); Hara, Osamu c/o MEIJI SEIKA KAISHA LTD, Kohhoku-ku Yokohama-shi Kanagawa-ken (JP); Miyado, Shinji c/o MEIJI SEIKA KAISHA LTD, Kohhoku-ku Yokohama-shi Kanagawa-ken (JP); Kumada, Yoichi c/o MEIJI SEIKA KAISHA LTD, Kohhoku-ku Yokohama-shi Kanagawa-ken (JP); Anzai, Hiroyuki c/o MEIJI SEIKA KAISHA LTD, Kohhoku-ku Yokohama-shi Kanagawa-ken (JP); Nagaoka, Kozo c/o MEIJI SEIKA KAISHA LTD, Kohhoku-ku Yokohama-shi Kanagawa-ken (JP)
(74) Representative: Sajda, Wolf E., Dipl.-Phys.

(56) References cited:
- EP-A- 0 068 497
- EP-A- 0 248 357
- GB-A- 2 161 159
- PATENT ABSTRACTS OF JAPAN, vol. 6, no. 118 (C-111)[996], 2nd July 1982

## Description

This invention relates to a new process for the production of L-2-amino-4-(hydroxymethylphosphinyl)-butyric acid which exhibits herbicidal activities and is known to be useful as a herbicide (see Japanese patent publication No. 56210/86 specification or U.S. patent No. 4 265 654 specification) as defined in the preamble of claim 1 (GB-A-2 161 159).

### Background of the Invention

The known methods for the production of L-2-amino-4-(hydroxymethylphosphinyl)-butyric acid (hereinafter abbreviated as "L-AMPB") represented by the formula (I): 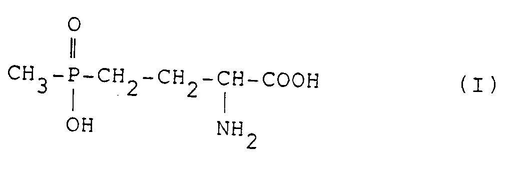
include such method wherein an antibiotic substance SF-1293 namely, L-2-amino-4-(hydroxymethylphosphinyl)-butyryl-L-alanyl-L-alanine, which contains L-AMPB as a moiety of the SF-1293 molecule and has herbicidal activities (also called "bialaphos"; see Japanese Patent Publication No. 639/76 and U.S. patent No. 4 309 208) is subjected to hydrolysis (see Japanese Patent Application first publication "Kokai" No. 85538/73), and such method wherein the SF-1293 substance is decomposed with a microbial enzyme (see Japanese Patent Application first publication "Kokai" No. 31890/74). Besides, there has also been known a further method wherein AMPB in the form of a racemic mixture is at first produced by a chemical synthetic process (see Japanese Patent Application first publication "Kokai" Nos. 91019/73 and 84529/79) and then the racemic AMPB product is subjected to optical resolution with aid of a microbial enzyme to yield L-AMPB. In addition, there has been known another method as reported recently by the present inventors, wherein an L-AMPB producing strain of the genus Streptomyces is cultivated, followed by recovering L-AMPB directly from the resulting culture broth (see Japanese Patent Application first publication "Kokai" No. 47485/82).

In order that a substance having herbicidal activities such as L-AMPB is produced, investigated and developed as a herbicide and then put actually on the market as a commercial herbicide product, some investigation needs to be made indispensably to improve the process of producing the herbicidal substance so as to make said process suitable for the low-price and large-scale production, while making considerable researches and development works for enhancing the safety and the herbicidal effects of said substance. The AMPB as produced by the above-described chemical synthetic process is a mixture of L-AMPB and D-AMPB. However, D-AMPB itself substantially lacks herbicidal activities. Moreover, D-AMPB is a non-natural substance and when applied to the soil, its decomposition by soil bacteria is slow so that it tends to remain in the soil and may cause environmental pollution in some instances. When L-AMPB is to be produced by the synthetic process, AMPB can be first produced in the form of a racemic mixture. It is hence necessary to isolate L-AMPB and D-AMPB separately from each other out of the racemic mixture. The synthetic process is therefore cumbersome and the yield of L-AMPB is low. In contrast, the process of producing L-AMPB which makes use of the microorganism or microbial enzyme can provide exclusively L-AMPB which is a naturally occurring substance. The naturally-occurring L-AMPB is considered to be an ideal herbicide which is free of the danger of environmental pollution, since such portions of the L-AMPB which have taken no part in effecting the herbicidal effects and are still remaining in the soil are easily decomposed and matabolyzed by soil bacteria and are hence not allowed to remain in the soil for long duration.

With the foregoing in view, we, the present inventors, have made researches in an attempt to combine the merit of the chemical synthetic process of producing AMPB that AMPB can be produced in a large scale, with the advantage of the microbiological process of producing L-AMPB that L-AMPB can exclusively be produced, so that an improved process for the production of L-AMPB capable of producing L-AMPB selectively in a large scale will be established.

L-AMPB can be deemed as a sort of derivatives of α-amino acid. Regarding such usual α-amino acids which normally constitute proteins, it has been known that α-amino acids can generally be formed by transamination of their corresponding 2-oxo-acids under the action of specific transaminases. We, the present inventors, have therefore been interesting in a 2-oxo-acid corresponding to L-AMPB, and have made an extensive investigation on such 2-oxo-acid. As a result, it has unexpectedly been discovered that when 4-(hydroxymethylphosphinyl)-2-oxo-butyric acid (hereinafter abbreviated as OMPB) is treated with a certain sort of transaminase or a microorganism capable of producing such transaminase, in the presence of at least one amino donor, OMPB can be converted into L-AMPB in a significant yield for a reasonable reaction time.

### Detailed Description of the Invention

According to this invention, therefore, there is provided a process for the production of L-2-amino-4-(hydroxymethylphosphinyl)-butyric acid represented by the formula (I): 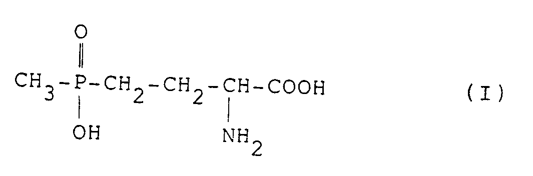
by treating 4-(hydroxymethylphosphinyl)-2-oxo-butyric acid represented by the formula (II): 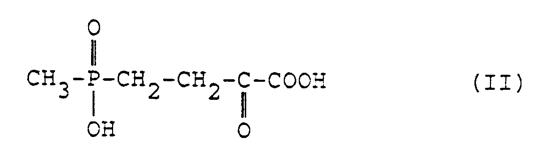
with glutamic acid or its salt as an amino-donor to the substrate, 4-(hydroxymethylphosphinyl)-2-oxo-butyric acid, in the presence of a transaminase which is capable of converting the substrate compound according to formula (II) into L-amino-4-(hydroxymethylphosphinyl)-butyric acid according to formula (I) in the presence of glutamic acid or its salt as the amino-donor and wherein the glutamic acid or its salt as the amino-donor is converted into 2-keto-glutaric acid or its salt as by-produced under the action of the transaminase, which is
characterized in that the transamination reaction of the substrate, 4-(hydroxymethylphosphinyl)-2-oxo-butyric acid according to formula (II) with glutamic acid or its salt as the amino-donor is effective in an aqueous reaction medium at an alkaline pH-value of 8,0 to 9,0 by reacting the substrate, 4-(hydroxymethylphosphinyl)-2-oxo-butyric acid at its initial high concentration with glutamic acid or its salt as added initially in a molar proportion of 0,2 to 3,0 mol per 1 mol of the substrate (OMPB) and in the presence of aspartic acid or its salt as added initially in a molar proportion of 1,0 to 3,0 mol per 1 mol of the substrate (OMPB) and in the presence of cultured cells of such a microorganism which is capable of producing a transaminase having an enzymatic activity to convert the substrate (OMPB) into the desired product, L-2-amino-4-(hydroxymethylphosphinyl)-butyric acid, in the presence of glutamic acid or its salt as the amino-donor and which is also capable of producing a transaminase having a GOT activity to regenerate the by-produced 2-keto-glutaric acid or its salt into glutamic acid or its salt in the presence of aspartic acid or its salt acting as a second amino-donor to give its amino group to the 2-keto-glutaric acid.

In a further development of the process according to the invention, the substrate compound (OMPB) is dissolved at its initial high concentration of 0,1 to 100 mg/ml in the aqueous reaction medium.

In a still further development of the process according to the invention, the transamination reaction is effected at a temperature in a range between room temperature and 60 °C.

Upon carrying out the process of this invention, the transaminase or the microorganism capable of producing transaminase is caused to react with OMPB and the amino-donor compound in an aqueous liquid reaction medium which contains OMPB and the compound capable of acting as the amino-donor dissolved therein.

In the process of this invention, it may generally be preferable to conduct the conversion reaction of OMPB into L-AMPB while adjusting the reaction medium at a pH in a range of 7,5 or higher, preferably, within a range of 8,0-9,0. The pH adjustment can be effected by adding sodium hydroxide or a suitable buffer solution. Desirable reaction conditions may be set so that the temperature and pH of the reaction medium fall within such temperature and pH ranges optimal for the action of the transaminase or the transaminase-producing microorganism participating in the reaction. Usually, it is preferable to conduct the reaction at a temperature in a range of from room temperature to 60°C preferably of 25°C to 50°C.

The OMPB used as the starting compound is a known substance. The production and physicochemical properties of OMPB are described, for example, in Japanese Patent Application first publication "Kokai" No. 92897/81 or U.S. patent No. 4 399 287 specification. In the process of this invention, the starting OMPB compound is usually dissolved in the reaction medium at an initial OMPB concentration preferably within a range of 0,10-100 mg/ml. at the beginning of the reaction.

According to a still further development of the process according to the invention, the microorganism used which is capable of producing the transaminase is selected from Streptomyces hygroscopicus SF-1293 FERM BP-130 or ATCC 21705, Streptomyces hygroscopicus NP-50 FERM BP-1368, Streptomyces lividans 66 FERM BP-737, Streptomyces albus IFO 13014 (ATCC 3004), Streptomyces griseus IFO 12875 (ATCC 23345), Streptovericillium cinnamoneum IFO 12852 (ATCC 11874), Streptomyces morookaensis IFO 13416 (ATCC 19166), Nocardia mediterranei ATCC 21271, Nocardiopsis dassonvillei JCM 3237, Streptomyces viridochromogenes IFO 13347 (ATCC 14920), Streptomyces viridochromogenes JCM 4977, Micromonospora carbonaceae NRRL 2972 (ATCC 27114), Escherichia coli ATCC 10798, Pseudomonas aeruginosa ATCC 10145, Pseudomonas cepacia ATCC 17759, Serratia marcescens ATCC 13880, and Mucor spinescens IAM Mu3.

Preferable examples of the microorganism available in the process of the process of this invention may include Streptomyces hygroscopicus SF-1293 strain (FERM BP-130 or ATCC 21705; see Japanese patent publication No. 639/76 or U.S. Patent No. 3,832,394 specification), which has been known as an SF-1293 substance-producing strain of Streptomyces, and its mutant strain, Streptomyces hygroscopicus NP-50 strain (FERM P-7804 or FERM BP-1368; see Japanese Patent Application first publication "Kokai" No. 58589/86 or European patent application publication No. 0 173 327) as well as Streptomyces lividans 66 strain (FERM BP-737; see Japanese Patent Application first publication "Kokai" No. 175889/84 or European patent application publication No. 0 196 375 specification). In addition, any other microorganisms can also be used so long as they can each produce an enzyme having the transaminase activity capable of converting OMPB into L-AMPB in the presence of the amino-donor. Microbiological characteristics of the Streptomyces hygroscopicus SF-1293 strain are described in Japanese Patent Publication No. 639/76 or U.S. Patent No. 3,832,394 specification. The Streptomyces hygroscopicus NP-50 strain (FERM BP-1368) has the same microbiological characteristics as those of the aforesaid SF-1293 strain but the genetic character of the NP-50 strain is different from that of the SF-1293 strain in that the NP-50 strain is lacking the biosynthetic ability of producing the SF-1293 substance (see Japanese patent Application first publication "Kokai" No. 58589/86 or European patent application publication No. 0 173 327). Further, the microbiological characteristics of Streptomyces lividans 66 strain (FERM BP-737) are described in the Japanese patent application first publication "Kokai" No. 175889/84. Among the above-mentioned microbial species, such those having the FERM P-numbers have been deposited and stored in a Japanese public depository, the "Fermentation Research Institute, Agency of Industrial Science & Technology, Ministry of International Trade and Industry", of 1-3, Higashi l-chome, Yatabe-machi, Tsukuba-gun, Ibaraki-ken, Japan, and such those having the FERM BP-number have been deposited and stored in the same Japanese public depository under the Budapest Treaty.

The enzyme useful in the process of this invention, i.e., the transaminase may be any transaminase so long as it possesses the transaminase activity capable of converting OMPB into L-AMPB in the presence of the amino-donor.

Preferred examples of the transaminase useful in the process of this invention may include a commercially available glutamic acid-oxaloacetic acid-transaminase (usually abbreviated as GOT) (International Enzyme Classification Number EC 2,6,1,1) and a commercially available glutamic acid-pyruvic acid-transaminase (usually abbreviated as GPT ) (International Enzyme Classification Number EC 2,6,1,2), as well as such enzymes having the transaminase activity capable of converting OMPB into L-AMPB in the presence of L-glutamic acid as the amino-donor. These transaminases may be used either singly or in combination of two or more of them. Of these, a preferred combination of the transaminases is a combination or system of a transaminase also having the enzymatic activity of converting 2-ketoglutaric acid into glutamic acid in the presence of aspartic acid as the amino-donor, namely such transaminase also having so-called GOT activity, and a second enzyme having the transaminase activity of converting OMPB into L-AMPB in the presence of glutamic acid as the amino-donor. For instance, it is possible to use, as such transaminase also having so-called GOT activity, the commercially-available glutamic acid-oxaloacetic acid-transaminase (International Enzyme Classification Number: EC 2,6,1,1); or such transaminase also having the GOT activity which has been extracted by a conventional method from a microorganism having the GOT activity, for example, Streptomyces hygroscopicus SF-1293 strain (see Japanese Patent Application first publication "Kokai" No. 47485/82; FERM BP-130; ATCC 21705). These transaminases also having the GOT activity may again be used either singly or in combination. As the second transaminase which can convert OMPB into L-AMPB in the presence of L-glutamic acid as the amino-donor and which is used in combination with said transaminase also having the so-called GOT activity, any transaminase may be employed as long as it can convert OMPB into L-AMPB in the presence of L-glutamic acid.

As the amino-donor compounds useful in the process of this invention, any known amino-donors such as those disclosed in the "SEIKAGAKU JIKKEN KOZA", Volume 11 "Metabolism of Amino Acids and Bio-amines", compiled by The Japanese Biochemical Society and published from Tokyo Kagaku Dozin Company Limited or in the "Journal of Biological Chemistry", 247, 2486 (1972) may all be used.

Preferred examples of the amino-donor compounds available according to this invention include straight-chain aliphatic L-α-amino acids such as L-glutamic acid, L-aspartic acid, L-alanine, L-methionine, L-glutamine and the like; branched-chain aliphatic L-α-amino acids such as L-leucine, L-isoleucine, L-valine and the like; basic amino acids such as L-lysine, L-ornithine, L-histidine and the like; as well as aromatic amino acids such as L-phenylalanine, L-tyrosine, L-tryptophan and the like, and an alkali metal salts such as sodium or potassium salt of these amino acids. Besides, D-amino acids corresponding to the above-mentioned L-amino acids may equally be used as the amino-donor in the present process. These amino-donors may be used either singly or in combination. As the amino-donor, it is preferable to use glutamic acid or a salt thereof, in combination with aspartic acid or a salt thereof. Molar ratio of the quantity of the amino donor to the quantity of OMPB present in the reaction medium may generally be in a range of 10:1 to 1:10 before the reaction is initiated.

When glutamic acid and/or aspartic acid is used as the amino-donor(s) in the process of this invention, commercially available glutamic acid or aspartic acid may be used as such. In general, these amino acids may each be in the form of a mixture of the D-isomer and L-isomer. Their L-isomers are however preferred. As salts of glutamic acid and aspartic acid, their alkali metal salts, especially, their sodium or potassium salts may be used.

In a preferred embodiment of the process of this invention, glutamic acid (or its salt) and aspartic acid (or its salt) are used in combination. The respective concentrations of these amino-donor compounds and the ratio of the amino-donor compounds to OMPB may preferably be set as described below. When these amino-donors are provided at higher ratios than OMPB so that a greater quantity of the amino donor is present than OMPB in the reaction medium, the reaction speed and the rate of the conversion of OMPB into L-AMPB can increase. Suitable ratio of the amino-donor compound to OMPB may, however, be set from the economical standpoint. In general, molar ratio of the concentration (or amount to be added) of glutamic acid or its salt to the concentration (or amount to be added) of OMPB may preferably be in a range of 0,2:1 to 3,0:1. On the other hand, the molar ratio of the concentration (or amount to be added) of aspartic acid or its salt to the concentration (or amount to be added) of OMPB may desirably be in a range of 1,0:1 to 3,0:1.

When a transaminase-producing microorganism is to be reacted with OMPB of the formula (II) and the amino-donor in the process of this invention, the present process may be carried out in such manner that said microorganism is at first cultured in a culture medium which contains nutrients useful in usual cultivation of microorganisms. It is possible to use, as the nutrient sources, any conventional nutrient sources which have been utilized in the ordinary cultivation of microorganisms. As the nutrient carbon source may be used, for example, glucose, starch, glycerin, sucrose, thick malt syrup, molasses or the like. They may be used either singly or in combination. As the nutrient nitrogen source may be used, for example, soybean meal, wheat germs, meat extract, peptone, dry yeast, corn steep liquor, ammonium sulfate, and the like, either singly or in combination. It is also feasible to add to the culture medium one or more inorganic salts such as calcium carbonate, sodium chloride, potassium chloride and phosphates as needed. As the cultivation method for the microorganism, the liquid cultivation method, especially, the submerged cultivation method is most suited. The cultivation of the microorganism may be conducted under aerobic conditions. The temperature suitable for the cultivation may range from 25°C to 40°C. The cultivation may suitably be continued for 1-4 days for actinomycetes, 1-2 days for bacteria, 1-2 days for yeasts and 1-4 days for fungi (molds).

The resulting culture broth of the transaminase-producing microorganism so cultivated may be used as such. If desired, the microbial cells may be separated from the culture broth and then washed with water or physiological saline. The resulting washed, intact cells may then be suspended at a suitable cell concentration in a volume of water, physiological saline or a suitable aqueous buffered solution to provide a cell suspension ready for use. To the culture broth or aqueous liquid suspension containing the cells of the microorganism employed, OMPB (a first substrate) and the amino-donor compound(s) (a second substrate for the enzyme) are then added either simultaneously or successively. The resultant liquid mixture is thereafter maintained under such conditions that the microorganism is allowed to react with OMPB and the amino-donor to proceed with the conversion reaction of OMPB into L-AMPB. The cell concentration and the concentrations of the OMPB and amino-donor in the aqueous liquid reaction medium, where OMPB and amino-donor are treated with the microorganism in the above-described manner to be converted into L-AMPB, as well as the reaction temperature and pH conditions may suitably be adjusted so as to maintain the reaction medium under such conditions that the conversion reaction of OMPB into L-AMPB should proceed efficiently and the microorganism should be allowed to exhibit its function. The reaction time may also be adjusted to produce and accumulate a substantial amount of L-AMPB in the reaction mixture.

When OMPB and the amino-donor compound are to be treated with transaminase(s) in the process of this invention on the other hand, OMPB and the amino-donor(s) are added and dissolved in an enzyme solution of suitable transaminase(s) in water or a buffered solution, followed by conducting the enzymatic reaction of OMPB. The respective concentrations of the transaminase(s), OMPB and amino-donor(s) in the reaction system as well as the reaction temperature and pH conditions may suitably be adjusted to their optimal ranges in which the conversion reaction of OMPB into L-AMPB should proceed with good efficiency.

As the transaminase may be used a transaminase which is available commercially in the form of an enzyme product. As an alternative, it is also feasible to use either such an aqueous, crude enzyme solution which has been prepared directly from a culture broth of an actinomycetes, bacterium, fungus or yeast as the transaminase-producing microorganism useful in the present invention by disintegrating the cells of the microorganism in said broth, or an aqueous solution of a crude enzyme product in water. The aqueous crude enzyme solution may also be used in the form of an aqueous extract of the disintegrated cells of the microorganism.

Such a crude enzyme solution which has been prepared from the above-described microorganism or its culture broth by a known method such as ultrasonic treatment or lysozyme treatment of the cells may also be used in the process of this invention, so long as said crude enzyme solution shows the ability to produce L-AMPB from OMPB in the presence of the amino-donor(s). Needless to say, an aqueous solution of the enzyme in a purified form may also be used. It is known that the stability and operation efficiency of an enzyme or microorganism can be enhanced by immobilizing same with aid of an organic solvent, crosslinking agent and carrier or the like. An immobilized transaminase or an immobilized, transaminase-producing microorganism, which has been treated by such a known immobilization method, may also be employed in the process of this invention as far as it has the ability to produce L-AMPB from OMPB.

In the process of this invention, the enzymatic reaction for converting OMPB into L-AMPB with transaminase(s) may be carried out preferably at a pH of 7,5 or higher, notably, within a pH range of 8,0-9,0. Reaction conditions should properly be chosen within the temperature and pH ranges optimal to the activity of the transaminase(s) which takes or take part in the enzymatic reaction.

In a preferred embodiment of the process of this invention, 4-(hydroxymethylphosphinyl)-2-oxo-butyric acid, namely, OMPB is treated with one or more transaminases or transaminase-producing microorganism(s) in the presence of both of L-glutamic acid or its salt and L-aspartic acid or its salt as the amino-donors. As the transaminase(s) employed in this embodiment, it is preferable to use an enzyme system which is composed of a combination of such an enzyme having the transaminase activity to convert 2-ketoglutaric acid into glutamic acid in the presence of L-aspartic acid as amino-donor, namely, such an enzyme having the transaminase activity which falls within the category of the so-called GOT activity, and such second enzyme having the transaminase activity to convert OMPB into L-AMPB in the presence of L-glutamic acid as amino-donor. Again, the microorganism to be employed in the above preferred embodiment of the present process may preferably be such a microorganism capable of producing an enzyme system which has not only an enzymatic activity to convert 2-ketoglutaric acid into glutamic acid in the presence of L-aspartic acid as amino-donor, i.e., the so-called GOT activity, but also a transaminase activity to convert OMPB into L-AMPB in the presence of L-glutamic acid as amino-donor, and said microorganism may be, for example, Streptomyces hygroscopicus SF-1293 strains (FERM BP-130 or ATCC 21705) or its mutant strain, Streptomyces hygroscopicus NP-50 strain (see Japanese Patent Application first publication "Kokai" No. 58589/86; FERM P-7804 or FERM BP-1368).

In the above preferred embodiment, it may be presumed that OMPB will receive the amino group from the glutamic acid (or its salt) as one of the amino-donors under the action of the above-mentioned transaminase system or microorganism so as to form L-AMPB with the glutamic acid turning to 2-ketoglutaric acid upon donation of its amino group, while the aspartic acid (or its salt) as the other amino-donor gives its amino group to said 2-ketoglutaric acid under the action of such transaminase also having the GOT activity, whereby 2-ketoglutaric acid can be regenerated into glutamic acid and aspartic acid (or its salt) itself is converted into oxaloacetic acid and finally into pyruvic acid.

In this way, the process of this invention provides a reaction solution containing L-AMPB as produced.

In a summary, therefore, the process of this invention may suitably be carried out in such a way that 4-(hydroxymethylphosphinyl)-2-oxo-butyric acid is treated or reacted with at least one transaminase in the presence of at least one amino-donor compound, preferably in the presence of both L-glutamic acid and L-aspartic acid or both their sodium salts, in an aqueous liquid reaction medium in which 4-(hydroxymethylphosphinyl)-2-oxo-butyric acid and the amino-donor compound(s) as well as the transaminase(s) have been dissolved, under alkaline conditions in a range of pH 7,5 to pH 9,0 and at a temperature in a range of room temperature to 60°C. Also, the process of this invention may suitably be carried out in such a way that 4-(hydroxymethylphosphinyl)-2-oxo-butyric acid is treated or reacted with at least one microorganisms capable of producing at least one transaminase, in the presence of at least one amino-donor compound, preferably in the presence of both L-glutamic acid and L-aspartic acid or both their sodium salts, in an aqueous liquid reaction medium in which 4-(hydroxymethylphosphinyl)-2-oxo-butyric acid and the amino-donor compound(s) have been dissolved and the cells of said microorganism have been suspended, under alkaline conditions of pH 7,5 to pH 9,0 and at a temperature in a range of room temperature to 60°C. When carrying out the present process using the cells of a transaminase-producing microorganism, 4-(hydroxymethylphosphinyl)-2-oxo-butyric acid and amino-donor compound(s) may be added to such a culture broth of a microorganism capable of producing the transaminase in which the intact cells of said microorganism are suspended, and then the interaction between 4-(hydroxymethylphosphinyl)-2-oxo-butyric acid, the amino-donor compound(s) and said microorganism is effected.

Again also, the process of this invention may suitably be carried out in such a way that 4-(hydroxymethylphosphinyl)-2-oxo-butyric acid is treated with an extract of a microorganism capable of producing at least one transaminase and containing the transaminase, in the presence of at least one amino-donor compound, preferably in the presence of both L-glutamic acid and L-aspartic acid or both their sodium salts, in an aqueous liquid reaction medium in which 4-(hydroxymethylphosphinyl)-2-oxo-butyric acid and the amino-donor compound(s) as well as the transaminase-containing extract of said microorganism have been dissolved, under alkaline conditions of pH 7,5 to pH 9,0 and at a temperature in a range of room temperature to 60°C. In whatever way the process of this invention is to be carried out, 4-(hydroxymethylphosphinyl)-2-oxo-butyric acid may be dissolved at its initial concentration of 0,1 to 100 mg/ml in an aqueous liquid reaction medium before the enzymatic reaction begins to proceed. And 4-(hydroxymethylphosphinyl)-2-oxo-butyric acid and the amino-donor compound(s) may be present at a molar ratio in a range of 1:10 to 10:1 in an aqueous liquid reaction medium initially before the enzymatic reaction of converting 4-(hydroxymethylphosphinyl)-2-oxo-butyric acid into L-2-amino-4-(hydroxymethylphosphinyl)-butyric acid takes place.

When the interaction between 4-(hydroxymethylphosphinyl)-2-oxo-butyric acid (i.e, OMPB), the amino-donor compound(s) and the transaminase(s) or the cells of the transaminase-producing microorganism has been effected according to the process of this invention, there is provided an aqueous reaction solution or mixture containing an amount of L-2-amino-4-(hydroxymethylphosphinyl)-butyric acid (i.e. L-AMPB) produced, an amount of OMPB remaining unreacted, an amount of the transaminase(s) used or the cells of the microorganism used. Before recovering L-AMPB from said reaction solution or mixture, it is preferable to centrifuge the reaction solution or mixture if this is containing the cells of the microorganisms used. By this centrifugation, the microbial cells can be removed from the reaction solution or mixture, and there is obtained a supernatant solution containing L-AMPB but free from the microbial cells.

Now, the recovery of the L-AMPB product from its aqueous solution is described below. Recovery and purification of L-AMPB from its aqueous solution so obtained may be conducted in the same manner as in the known methods of recovering and purifying L-AMPB from the culture broth of the L-AMPB-producing microorganism which is obtained according to the known fermentative method of producing L-AMPB. Detailed procedures for recovery and purification of L-AMPB are disclosed in Japanese patent application first publication "Kokai" No. 47485/82. For instance, the recovery and purification of L-AMPB from the reaction solution as obtained from the process of this invention may be achieved by passing the L-AMPB-containing reaction solution through a column of a cation-exchange resin such as Dowex 50 W (product of Rohm & Haas Co., Ltd., U.S.A.) to make L-AMPB adsorbed by this resin, and then eluting the resin column with water or diluted aqueous ammonia solution to give such fractions of the eluate containing L-AMPB. The eluate fractions containing L-AMPB may then been collected together and concentrated under reduced pressure to afford a dry powder of L-AMPB. The L-AMPB as produced according to the process of this invention shows the same physical and chemical properties as those of an authentic sample of L-AMPB which has been afforded by the known fermentative method as disclosed in the Japanese patent application first publication "Kokai" No. 47485/82, and also it has been observed that the L-AMPB as produced according to this invention shows the same herbicidal activities as those of the L-AMPB as produced by the known fermentative method tested by some herbicidal methods.

This invention is now illustrated with reference to the following Examples to which this invention is not limited.

### Example 1

The production of L-AMPB was conducted by reaction of different kinds of commercially available transaminases with OMPB which was dissolved in a volume of a 50 mM phosphate buffered solution (pH 6) containing an amino-donor compound as added.

Thus, in the experiments where glutamic acid-oxaloacetic acid-transaminase (GOT) (a product of Boehringer Mannheim Co., Ltd.) was employed as the transaminase enzyme, L-aspartic acid (Asp) and L-glutamic acid (Glu) were both added as the amino-donors (as the substrates for the donation of amino group) to and dissolved in the reaction medium. When glutamic acid-pyruvic acid-transaminase (GPT) (a product of Boehringer Mannheim Co., Ltd.) was used on the other hand, L-alanine (Ala) was added and dissolved as the amino-donor into the reaction medium. When glutamic acid dehydrogenase (GLDH) was employed for a reference test, ammonium chloride and NADH were both added as the amino-donor. For comparison purpose, production of glutamic acid (Glu) was also observed by reacting 2-ketoglutaric acid (2-KG) (as a control substrate) with the transaminase employed.

After conducting the enzymatic reaction at 30°C for 50 minutes in each experiment, the reaction mixture was heated at 100°C for 3 minutes to terminate the reaction. The reaction mixture or solution was adjusted to pH 2 by addition of diluted aqueous sulfuric and centrifuged to obtain a supernatant solution. The amounts of L-AMPB and glutamic acid as formed and present in the supernatant solution were determined by an amino acid analyzer. Results of the experiments are shown in Table 1.

**Table 1**

| Enzyme used and its amount added | Substrate compound and its concentration (µg/ml) | | Amino-donor compound and its concentration (µg/ml) | | Reaction product and its amount produced (µg/ml) | |
|---|---|---|---|---|---|---|
| GOT (10 units/ml) | OMPB | 100 | Asp | 1000 | L-AMPB | 2,8 |
| | OMPB | 100 | Glu | 1000 | L-AMPB | 3,7 |
| | 2-KG (Control) | 100 | Asp | 1000 | Glu | 109,3 |
| GPT (4 units/ml) | OMPB | 100 | Ala | 1000 | L-AMPB | 0,9 |
| | 2-KG (Control) | 100 | Ala | 1000 | Glu | 92,7 |
| GLDH (60 units/ml) | OMPB | 100 | NADH+NH₄Cl | 1000 | L-AMPB | 1,9 |
| | 2-KG (Control) | 100 | NADH+NH₄Cl | 1000 | Glu | 109,3 |

It is clear from the results of Table 1 that L-AMPB was produced from OMPB by the enzymatic reaction, although the yield of L-AMPB was significant but not so high as the yield of glutamic acid as produced from 2-ketoglutaric acid.

### Example 2

Test bacterial strains indicated in Table 2 given below were separately inoculated in 40-ml portions of a culture medium comprising Nutrient broth (product of Difco Corporation), followed by productive cultivation of the bacteria at 28°C for 6 hours. The resultant culture broths each were used as a seed culture, and it was inoculated at an inoculum size of 2% in 40-ml portions of a culture medium of the same composition as above and cultivated at 28°C overnight, and OMPB was added to a concentration of 100 µg/ml in each of the resulting culture broths containing the bacterial cells.

To each of the resulting culture broths containing OMPB as added, there was further added sodium L-aspartate to a concentration of 100 µg/ml as the amino-donor. The liquid mixtures comprising the culture broth, OMPB and sodium L-aspartate were thus provided. Thereafter, the enzymatic conversion of OMPB into L-AMPB was allowed to proceed at 28°C for 24 hours, and the reaction mixtures were adjusted to pH 2 with 25% sulfuric acid to terminate the reaction. The cells were removed by centrifugation. The amounts of L-AMPB as formed in the respective supernatant solutions obtained were determined using an amino acid analyzer. The test results are summarized in Table 2.

**Table 2**

| Tested bacterial strain | Amount of L-AMPB produced (µg/ml) | GOT activity (potency) of Crude enzyme solution (x 10⁻³ units/ml) | Specific GOT activity of enzyme (x 10⁻³ units/mg of protein) |
|---|---|---|---|
| Escherichia coli ATCC 10798 | 0,8 | 3,8 | 5,4 |
| Pseudomonas aeruginosa ATCC 10145 | 8,3 | 342,0 | 686,7 |
| Serratia marcescens ATCC 13880 | 5,0 | 45,6 | 300,0 |

It is apparent from Table 2 that AMPB can be produced from OMPB in significant yields.

Further, portions of the culture broths as obtained after the above-mentioned overnight cultivation at 28°C were separately subjected to an ultrasonic treatment to disintegrate the bacterial cells and obtain cell extracts. The cell extracts so prepared were separately centrifuged to produce aqueous crude enzyme solutions. The GOT activity levels (potency) of the respective crude enzyme solutions so obtained were also measured and are shown in Table 2 for information. As shown in Table 2, significant correlation is observed between the amounts of L-AMPB produced and the measured value of the GOT activity of said crude enzyme solution. Besides, said crude enzyme solutions were each analysed for their protein contents according to the Bio-Rad Protein Assay method, and the specific GOT activity of the enzyme (x 10⁻³ units/mg of protein) present in said solution was evaluated and shown in Table 2 above.

### Example 3

Streptomyces hygroscopicus SF-1293 strain (FERM BP-130) was inoculated in 10 ml of a preliminary culture medium (comprising 2,0% soluble starch, 1,0% polypeptone, 0,3% meat extract, 0,05% dipotassium hydrogen phosphate; pH 7.0). The above strain was shake-cultivated at 28°C for 24 hours. The resulting culture broth was used as a seed culture, and it was inoculated at an inoculum size of 2% into a productive culture medium (comprising 7,0% glucose, 4,4% bactosoyton, 0,327% potassium dihydrogen phosphate, 0,0852% disodium hydrogen phosphate, 1,15% dotite TES, namely N-tris(hydroxymethyl)methyl-2-amino-ethanesulfonic acid, 0.0001% cobalt chloride; pH 6,0) and then the cultivation of the SF-1293 strain was made at 28°C under aeration and agitation. After the cultivation for 4 days, the microbial cells were collected by centrifugation and washed with a 50mM phosphate buffered solution (pH 6,0). The cells were then disintegrated by ultrasonic treatment (using a device designated "KUBOTA INSONATOR"; 1,5A, for 1 minute), followed by centrifugation to obtain a crude enzyme solution.

OMPB was added to a concentration of 100 µg/ml and L-aspartic acid was added to a concentration of 200 µg/ml into said crude enzyme soltution. A further control test was also made using 2-ketoglutaric acid (2-KG) as a control substrate for the transamination reaction. After allowing the enzymatic reaction to proceed at 30°C for 2 hours, the reaction solutions were heated at 100°C for 3 minutes to terminate the reaction. The reaction solution obtained was adjusted to pH 2 with diluted sulfuric acid and a supernatant solution was obtained by centrifugation of the reaction solution. The amounts of L-AMPB and the glutamic acid as formed from 2-KG which were present in the supernatant solutions were determined by an amino acid analyzer. Test results are shown in Table 3.

**Table 3**

| Substrate | Product and its amount produced (µg/ml) | |
|---|---|---|
| OMPB | L-AMPB | 2.8 |
| 2-KG (Control) | Glu | 67.9 |

### Example 4

(1) Streptomyces hygroscopicus NP-50 strain FERM BP-1368) was inoculated in 10 ml of a preliminary culture medium (comprising 2,0% soluble starch, 1,0% polypeptone, 0,3% meat extract, 0,05% dipotassium hydrogen phosphate; pH 7,0). The above NP-50 strain was shake-cultured at 28°C for 24 hours. The resulting culture broth was used as a seed culture, and it was inoculated at an inoculum size of 2% into a productive culture medium (comprising 7,0% glucose, 4,4% bactosoyton, 0,327% potassium dihydrogen phosphate, 0,0852% disodium hydrogen phosphate, 1,15% dotite TES, 0,0001% cobalt chloride; pH 6,0) and then the cultivation of the NP-50 strain was made at 28°C under aeration and agitation.
(2) In a 250-ml Erlenmeyer flask, were combined 20 ml of the culture broth of Streptomyces hygroscopicus NP-50 strain cultivated for 3 days in the above productive culture medium, 30 ml of an aqeuous solution of OMPB (OMPB concentration: 87 mg/ml, adjusted to pH 7,0 in advance), 40 ml of an aqueous solution of commercial sodium L-glutamate (sodium L-glutamate concentration: 170 mg/ml) and 10 ml of 1M tris-HCl buffered solution (pH 8,5). The OMPB concentration in the combined liquids amounted to about 26 mg/ml. While gently shaking the resulting liquid mixture at 37°C in the flask, the enzymatic reaction was allowed to proceed for 24 hours. Thereafter, the reaction mixture so obtained was centrifuged to remove the microbial cells therefrom, and the resulting supernatant solution containing L-AMPB produced (100 ml) was analyzed by an amino acid analyzer to determine the amount of L-AMPB in the solution. It was found that 14 mg/ml of L-AMPB had been produced.
   In addition, similar experiments were also conducted using L-alanine or sodium L-aspartate separately as the amino-donors (as added to a concentration of 170 mg/ml). It was observed that 2,8 mg/ml of L-AMPB was produced when L-alanine was used, and 1,5 mg/ml of L-AMPB when sodium L-aspartate was employed.
(3) The supernatant solution (100 ml) obtained by centrifugating the reaction mixture which had been formed upon the use of sodium L-glutamate as the amino-donor in the above procedure (2) was passed into a column of 400 ml of a cation exchange resin "Dowex 50 W x 2" (H⁺-form) (trade name; product of Rohm & Haas Co.), followed by development with dilute aqueous ammonia. The L-AMPB-containing fractions of the eluate were collected and concentrated. The concentrated solution was then subjected to chromatography on an anion-exchange resin column of 150 ml of "Dowex 1 x 2" (CH₃COO⁻-form)(trade name; product of Rohm & Haas Co.). After washing the resin column with water, the column was eluted with a 0.3N aqueous solution of acetic acid.

The L-AMPB-containing fractions of the eluate were concentrated to dryness under reduced pressure, followed by drying in vacuo to give 720 mg of L-AMPB as a white powder. The white powder was recrystallized from methanol. The crystals thus obtained were analyzed in a usual manner, i.e., by determining the elemental analysis, specific optical rotation, melting point, infrared absorption spectrum, nuclear magnetic resonance spectrum and mass spectrum. It was confirmed that the crystals of L-AMPB obtained was fully indentical to an authentic sample of L-AMPB.

### Example 5

(1) The culture broth (20 ml) of Streptomyces hygroscopicus NP-50 strain (FERM BP-1368), which had been cultivated in the productive culture medium used in the procedure (1) of Example 4, was placed in a 30-ml centrifuge tube and then centrifuged at 3000 rpm. for 10 minutes. Precipitated cells of the NP-50 strain were suspended in 30 ml of 50mM tris-HCl buffered solution (pH 8.5) to prepare the cell suspension. In a 250-ml Erlenmeyer flask, were combined 20 ml of the cell suspension, 30 ml of an aqueous solution of OMPB (OMPB concentration: 87 mg/ml, adjusted to pH 7,0 in advance), 40 ml of an aqueous solution of commercial sodium L-glutamate (sodium L-glutamate concentration: 170 mg/ml) and 10 ml of 1M tris-HCl buffered solution (pH 8,5). The OMPB concentration in the combined liquids amounted to about 26 mg/ml. While gently shaking the resulting liquid mixture at 37°C, the enzymatic reaction was allowed to proceed for 24 hours. Thereafter, the reaction mixture so obtained was centrifuged to remove the microbial cells therefrom, and the resulting supernatant solution containing L-AMPB produced (100 ml) was analyzed by an amino acid analyzer to determine the amount of L-AMPB in the solution. It was found that 15 mg/ml of L-AMPB had been produced.
(2) In the same manner as in the procedure (3) of Example 4, L-AMPB-containing supernatant solution (100 ml) obtained in the above procedure (1) was subjected to chromatography on a column of 400 ml of a cation-exchange resin "Dowex 50W x 2" (H⁺-form), followed by development with a dilute aqueous ammonia. Upon posttreatment of the L-AMPB-containing fractions of the eluate in the same manner as in the procedure (3) of Example 4, 750 mg of L-AMPB was obtained as a white powder.

### Example 6

(1) The culture broth (20 ml) of Streptomyces hygroscopicus NP-50 strain (FERM BP-1368), which had been cultured in the productive culture medium used in the procedure (1) of Example 4, was placed in a 30-ml centrifuge tube and centrifuged at 3000 rpm. for 10 minutes. Precipitated cells of the NP-50 strain were suspended in 30 ml of 50mM tris-HCl buffered solution (pH 8,5) to prepare the cell suspension. After disintegrating the cells in the cell suspension for 10 minutes by an ultrasonic disintegrator, the resultant suspension was centrifuged at 10000 rpm. for 10 minutes to yield 20 ml of a crude enzyme solution as the supernatant solution.
(2) In a 250-ml Erlenmeyer flask, were combined 20 ml of the supernatant solution (the crude enzyme solution) obtained as above, 30 ml of an aquous solution of OMPB (OMPB concentration: 87 mg/ml, adjusted to pH 7,0 in advance), 40 ml of an aqueous solution of commercial sodium L-glutamate (sodium L-glutamate concentration: 170 mg/ml) and 10 ml of 1M tris-HCl buffered solution (pH 8.5). The OMPB concentration in the combined liquids amounted to about 26 mg/ml. While gently shaking the resulting liquid mixture at 37°C, the enzymatic reaction was allowed to proceed for 24 hours. Thereafter, the resulting reaction mixture obtained (100 ml) was analyzed by an amino acid analyzer to determine the amount of L-AMPB formed in said reaction mixture. It was found that 16 mg/ml of L-AMPB had been produced.
(3) In the same manner as in the procedure (3) of Example 4, the reaction mixture (100 ml) obtained from the enzymatic reaction of the above procedure (2) was subjected to chromatography on an ion-exchange resin column of 400 ml of "Dowex 50W x 2" (H⁺-form), followed by development with a dilute aqueous ammonia. Upon posttreatment of the L-AMPB-containing fractions of the eluate in the same manner as in the procedure (3) of Example 4, 740 mg of L-AMPB was obtained as a white powder.

### Example 7

(1) Streptomyces lividans 66 strain (FERM BP-737) was inoculated in 10 ml of a preliminary culture medium of the same composition as that employed in the procedure (1) of Example 4. The above microorganism was shake-cultivated at 28°C for 24 hours. The resultant culture broth was used as a seed culture, and it was inoculated at an inoculum size of 2% in a productive culture medium of the same composition as that used in the procedure (1) of Example 4 and was then cultivated at 28°C under aeration and agitation.
(2) In a 250-ml Erlenmeyer flask, were combined 20 ml of the culture broth of Streptomyces lividans 66 strain cultivated for 3 days in the above productive culture medium, 30 ml of an aqueous solution of OMPB (OMPB concentration: 87 mg/ml, adjusted to pH 7,0 in advance), 40 ml of an aqueous solution of commercial sodium L-glutamate (sodium L-glutamate concentration: 170 mg/ml) and 10 ml of 1M tris-HCl buffered solution (pH 8,5). The OMPB concentration in the combined liquids amounted to about 26 mg/ml. While gently shaking the resulting liquid mixture at 37°C in the flask, the enzymatic reaction was allowed to proceed for 24 hours. Thereafter, the reaction mixture so obtained was centrifuged to remove the microbial cells therefrom, and the resulting supernatant solution containing L-AMPB produced (100 ml) was analyzed by an amino acid analyzer to determine the amount of L-AMPB in the supernatant solution. It was found that 6 mg/ml of L-AMPB had been produced.

### Example 8

(1) Streptomyces hygroscopicus SF-1293 strain (FERM BP-130; ATCC 21705) was inoculated in 10 ml of a preliminary culture medium of the same composition as that employed in the procedure (1) of Example 4. The above SF-1293 strain was shake-cultivated at 28°C for 24 hours. The resultant culture broth was used as a seed culture, and it was inoculated at an inoculum size of 2% in a productive culture medium of the same composition as that used in the procedure (1) of Example 4 and then the SF-1293 strain was cultivated at 28°C under aeration and agitation.
(2) In a 250-ml Erlenmeyer flask, were combined 20 ml of the culture broth of Streptomyces hygroscopicus SF-1293 strain cultured for 3 days in the above productive culture medium, 30 ml of an aqueous solution of OMPB (OMPB concentration: 87 mg/ml, adjusted to pH 7,0 in advance), 40 ml of an aqueous solution of commercial sodium L-glutamate (sodium L-glutamate concentration: 170 mg/ml) and 10 ml of 1M tris-HCl buffered solution (pH 8,5). The OMPB concentration in the combined liquids amounted to about 26 mg/ml. While gently shaking the resulting liquid mixture at 37°C in the flask, the enzymatic reaction was allowed to proceed for 24 hours. Thereafter, the reaction mixture so obtained was centrifuged to remove the microbial cells therefrom, and the resulting supernatant solution containing L-AMPB produced (100 ml) was analyzed by an amino acid analyzer to determine the amount of L-AMPB produced in the supernatant solution. It was found that 7 mg/ml of L-AMPB had been produced.
(3) In the same manner as in the procedure (3) of Example 4, the supernatant solution (100 ml) obtained in the above procedure (2) was subjected to chromatography on an ion-exchange resin column of 400 ml of "Dowex 50W x 2" (H⁺-form), followed by development with a dilute aqueous ammonia. Upon post-treatment of the L-AMPB-containing fractions of the eluate in the same manner as in the procedure (3) of Example 4, 350 mg of L-AMPB was obtained as a white powder.

### Example 9

Suspended in 100 ml of 50mM tris-HCl buffered solution (pH 8,5) was 20 g of a wet cake of commercial baker's yeast (Saccharomyces cerevisae; a procuct of Oriental Yeast Co., Japan). In a 250-ml Erlenmeyer flask, were combined 20 ml of the resulting cell suspension of the baker's yeast, 30 ml of an aqueous solution of OMPB (OMPB concentration: 87 mg/ml, adjusted to pH 7,0 in advance), 40 ml of an aqueous solution of commercial sodium L-glutamate (sodium L-glutamate concentration: 170 mg/ml) and 10 ml of 1M tris-HCl buffered solution (pH 8,5). The OMPB concentration in the combined liquids then amounted to about 26 mg/ml. While shaking the resulting liquid mixture at 37°C for 24 hours, the enzymatic reaction of OMPB was allowed to proceed. The reaction mixture so obtained was centrifuged to remove the yeast cells therefrom, and the resulting supernatant solution containing L-AMPB produced (100 ml) was analyzed by an amino acid analyzer to determine the amount of L-AMPB present in the supernatant solution. It was found that 6 mg/ml of L-AMPB had been produced.

### Example 10

In 50 mM tris-HCl buffered solution (pH 8,5) containing 400 µg/ml of OMPB and 600 µg/ml of L-glutamic acid added and dissolved therein, a transaminase was reacted with OMPB to produce L-AMPB. As this transaminase, a commercial glutamic acid-oxaloacetic acid-transaminase (GOT) (a product of Boehringer Mannheim Co.) was used. The GOT concentration was set at 40 units/ml. After allowing the enzymatic reaction to proceed at 37°C for 24 hours, the resulting reaction solution was heated at 100°C for 3 minutes to terminate the reaction. After this, the reaction solution was adjusted to pH 2 with diluted sulfuric acid and then centrifuged to remove the insoluble solids and give a supernatant solution. The amount of L-AMPB in the supernatant solution was determined by an amino acid analyzer. The amino acid analyzer was Model "MLC-703" manufactured by ATTO CORPORATION, Japan, and the retention time was set for 12 minutes. It was found that 100 µg/ml of L-AMPB had been produced in the supernatant solution.

### Example 11

### (1) Cultivation method:

Several species of microorganisms indicated in Tables 4 to 8 below were separately inoculated from their respective seed culture slants into volumes (10 ml-portions) of a liquid culture medium (comprising 0,5% glucose, 0,3% yeast extract, 1,0% meat extract, 1,0% peptone, 0,3% sodium chloride; pH 7,0) as charged in large-capacity test tubes. A piece of stainless steel-coil was also placed in such test tubes as employed for the cultivation of the actinomycetes. The inoculated microorganisms were then separately cultivated at 28°C for 24 hours (or 48 hours) on tube shakers.

### (2) Preparation of cell samples:

After the cultivation, 1-ml portions of the resulting culture broths (except 0,2-ml portions of the fungi culture in the case of the fungi) were each withdrawn into micro-test tubes (product of Eppendorf Co., Ltd.) and centrifuged at 12000 rpm. for 3 minutes. The supernatants as formed were discarded and the remaining cells deposited were each collected as the cell samples.

### (3) Engymatic reactions:

Into each of the cell samples so packed in the respective micro-test tubes, were then added 20 µℓ of tris-HCl buffered solution (1 mole, pH 8,0), 20 µℓ of an aqueous OMPB solution (OMPB concentration: 200 mg/ml, pH 8,0), 40 µℓ of an aqueous solution of an amino-donor as indicated below (containing either one of sodium L-glutamate, sodium L-aspartate and L-alanine, at a concentration of 1 mol/ml) and 120 µℓ of water. The resultant mixture was allowed to stand at 37°C for 24 hours to the effect the interaction between the microbial cells, OMPB and the amino-donor compound present. After the reaction, the reaction mixture was centrifuged at 12000 rpm. for 3 minutes, and the amount of L-AMPB present in the supernatant solution obtained was measured by determining the amount of L-AMPB with an amino acid analyzer. Test results are summarized in Tables 4 to 8.

**Table 4**

| Microorganisms used (Bacteria) | Amount of L-AMPB produced (mg/ml) | | |
|---|---|---|---|
| | Glu | Asp | Ala |
| Escherichia coli ATCC 10798 | 9,8 | 4,2 | 3,3 |
| Pseudomonas aeruginosa ATCC 10145 | 8,3 | 3,3 | 3,8 |
| Pseudomonas cepacia ATCC 17759 | 9,6 | 2,4 | 3,0 |
| Serratia marcescens ATCC 13880 | 9,5 | 1,8 | 1,6 |

**Table 5**

| Microorganisms used (Actinomycetes) | Amount of L-AMPB produced (mg/ml) | | |
|---|---|---|---|
| | Glu | Asp | Ala |
| Streptomyces albus IFO 13014 (ATCC 3004) | 7,4 | 1,0 | 0,8 |
| Streptomyces griseus IFO 12875 (ATCC 23345) | 8,3 | 1,7 | 1,9 |
| Streptovericillium cinnamoneum IFO 12852 (ATCC 11874) | 10,6 | 6,0 | 2,2 |
| Streptomyces morookaensis IFO 13416 (ATCC 19166) | 6,8 | 7,5 | 3,3 |
| Nocardia mediterranei ATCC 21271 | 8,1 | 2,6 | 1,6 |
| Nocardiopsis dassonvillei JCM 3237 | 6,3 | 1,5 | 1,5 |

**Table 6**

| Microorganisms used (Actinomycetes) | Amount of L-AMPB produced (mg/ml) | | |
|---|---|---|---|
| | Glu | Asp | Ala |
| Streptomyces viridochromogenes IFO 13347 (ATCC 14920) | 9,5 | 1,6 | 1,1 |
| Micromonospora carbonaceae NRRL 2972 (ATCC 27114) | 6,1 | 1,4 | 1,6 |
| * Cultivation time: 48 hours | | | |

**Table 7**

| Microorganism used (Actinomycetes) | Amount of L-AMPB produced (mg/ml) | | |
|---|---|---|---|
| | Glu | Asp | Ala |
| Streptomyces viridochromogenes JCM 4977 * | 8,8 | 5,6 | 3,6 |

| | | | |
|---|---|---|---|
| * Bialaphos-producing actinomycetes | | | |

**Table 8**

| Microorganisms used (Fungi) | Amount of L-AMPB produced (mg/ml) | | |
|---|---|---|---|
| | Glu | Asp | Ala |
| Mucor spinescens IAM Mu3 | 5,3 | 0,9 | 1,1 |
| * Cultivation time: 48 hours | | | |

Amongst the species or strains of the microorganisms indicated in Table 2 and Tables 4 to 8 hereinbefore,the species or strains having the ATCC-numbers have been deposited and stored in the "American Type Culture Collection", Washington D.C., U.S.A.; the species or strains having the JCM-numbers have been deposited and stored in the "Japan Collection of Microorganism", Insitute of Physical and Chemical Research at Wako City, Saitama-ken, Japan; the species or strains having the IAM-numbers have been deposited and stored in the "Institute of Applied Microbiology, University of Tokyo", at Tokyo, Japan; and the species or strains having the IFO-numbers have been deposited and stored in the "Institute for Fermentation, Osaka" at Osaka, Japan. All of the species or strain of the microorganisms having the access numbers indicated as above are available and distributable as the known Type Culture strains from the above-identified public depositories.

### Example 12

(1) Streptomyces hygroscopicus NP-50 strain (FERM BP-1368) was inoculated in 10 ml of a prelimainary culture medium (comprising 2,0% soluble starch, 1,0% polypeptone, 0,3% meat extract, 0,05% dipotassium hydrogen phosphate; pH 7,0). The above NP-50 strain was shake-cultured at 28°C for 24 hours. The resultant culture broth was used as a seed culture, and it was inoculated at an inoculum size of 2% in a productive culture medium (comprising 7,0% glucose, 3,9% wheat germs, 2,5% soluble vegetative protein, 0,3% potassium dihydrogen phosphate, 0,0001% cobalt chloride; pH 6,8). The cultivation was then conducted at 28°C under aeration and stirring.
(2) To 20-ml portions of the culture broth obtained by cultivation of the NP-50 strain in the above mentioned productive culture medium for 3 days and containing cells of Streptomyces hygroscopicus NP-50 strain, were added OMPB and sodium L-glutamate and sodium L-aspartate as the amino-donors in such quantities so as to give their respective concentrations in the resulting mixture as given in Table 9 below at the beginning of the respective transamination reaction. By further addition of 10 ml of 1M tris-HCl buffered solution (pH 8,5), each of the resulting mixtures was adjusted to pH 8,5 and the volume of the resultant mixture was brought to 100 ml. Thus, the original volume of said culture broth had been diluted fivefold.

The enzymatic amination reaction of OMPB was then allowed to proceed at 37°C for 24 hours with gently shaking the resultant solution containing the cells suspended therein as well as OMPB and the amino-donor compounds dissolved therein.

After the reaction, 100-ml portions of the respective reaction mixtures each were subjected to a heat treatment (sterilization and inactivation of the enzyme) so as to terminate the reaction. The reaction mixture each were filtered to remove the microbial cells, and the amounts of L-AMPB in the individual filtrates obtained were measured by an amino acid analyzer ( " MLC-703", trade name, manufactured by ATTO CORPORATION: retention time of 12 minutes). Test results are shown in Table 9.

**Table 9**

| Test No. | Initial Concentration of OMPB (µg/ml) | Initial concentration of amino donor before start of reaction (µg/ml) | | Final concentration of L-AMPB produced after end of reaction (µg/ml) |
|---|---|---|---|---|
| | | Sodium L-glutamate | Sodium L-aspartate | |
| 1 | 10000 | 10000 | 0 | 5930 |
| 2 | 10000 | 20000 | 0 | 7170 |
| 3 | 10000 | 0 | 10000 | 350 |
| 4 | 10000 | 10000 | 10000 | 8500 |
| 5 | 30000 | 30000 | 30000 | 28360 |

From the results of Table 9, it is clear that the amount of L-AMPB produced from OMPB increased significantly when the enzymatic amination reaction of OMPB was effected in the presence of sodium L-glutamate and sodium L-aspartate in combination as the amino-donors, as compared to when the enzymatic reaction of OMPB was effected in the presence of either one of sodium L-glutamate and sodium L-aspartate.

### Example 13

A culture broth of Streptomyces hygroscopicus NP-50 strain, which had been prepared under the same preliminary cultivation conditions as in Example 12, was used as a seed culture, and it was inoculated into a volume of the same productive culture medium as that employed in Example 12 in a 3-literes jar fermentor, followed by conducting the cultivation at 28°C for 3 days.

Two liters of the culture broth so obtained was transferred into a reaction tank, followed by addition of 300 g of OMPB into the culture broth. In addition, 310 g of sodium L-glutamate (product of Wako Pure Chemical Industries, Ltd.) and 290 g of sodium L-aspartate (product of Nakarai Chemicals, Ltd.) were also added and dissolved in said culture broth. After adjusting the resultant mixture to pH 8,5 with aqueous NaOH, the volume of the mixture was brought to 10 liters by addition of water. Thus, the concentration of OMPB in the resulting liquid mixture was 30000 µg/ml at the start of the reaction. The respective concentrations of sodium L-glutamate and sodium L-aspartate were equal at this time to the molar concentration of OMPB (1/6 mole). The enzymatic amination reaction of OMPB was then allowed to proceed at 37°C for 24 hours with gently stirring the liquid mixture containing the cells of the NP-50 strain, OMPB and the amino-donor compounds, and with controlling the pH at 8,5. After the reaction, the reaction mixture obtained was adjusted to pH 3,0 with addition of 50% sulfuric acid. After addition of a filter aid, the reaction mixture was filtered to remove the cells, affording 9 ℓ of the filtrate.

The concentration of L-AMPB in the filtrate was analyzed in the same manner as in Example 12. It was found to be 29000 µg/ml. The filtrate was then chromatographed by passing through a column of 5 ℓ of "Dowex 50 W" (H⁺-form) as a cation-exchange resin, followed by development with water. The L-AMPB containing fractions of the eluate were again subjected to the chromatography on a column of "Dowex 1 x 2"(CH₃COO⁻-form) as an anion-ion exchange resin, which was washed with water and then eluted with a 0,3N aqueous solution of acetic acid.

The L-AMPB-containing fractions of the eluate were concentrated to dryness under reduced pressure, followed by drying in vacuo to give L-AMPB as white powder. This white powder was recrystallized from methanol. The yield of L-AMPB was 183 g (about 70%).

The crystals of L-AMPB thus obtained were analyzed in a usual manner, i.e., by measuring the elemental analysis, specific optical rotation, melting point, infrared absorption spectrum, nuclear magnetic resonance spectrum and mass spectrum. It was confirmed that the L-AMPB crystal obtained was fully identical to an authentic sample of L-AMPB.

## Claims

1. A process for the production of L-2-amino-4-(hydroxymethylphosphinyl)-butyric acid of formula (I): 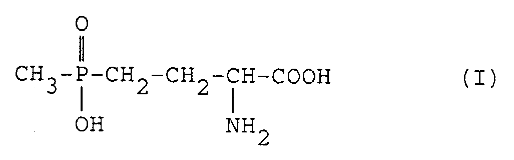 by reacting 4-(hydroxymethylphosphinyl)-2-oxo-butyric acid of formula (II): 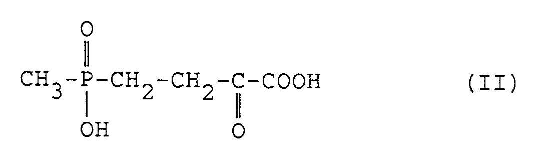 with glutamic acid or its salt as an amino-donor to the substrate, 4-(hydroxymethylphosphinyl)-2-oxo-butyric acid, in the presence of a transaminase which is capable of converting the substrate compound according to formula (II) into L-amino-4-(hydroxymethylphosphinyl)-butyric acid according to formula (I) in the presence of glutamic acid or its salt as the amino-donor and wherein the glutamic acid or its salt as the amino-donor is converted into 2-keto-glutaric acid or its salt as by-produced under the action of the transaminase,
characterized in that the transamination reaction of the substrate, 4-(hydroxymethylphosphinyl)-2-oxo-butyric acid according to formula (II) with glutamic acid or its salt as the amino-donor is effective in an aqueous reaction medium at an alkaline pH-value of 8,0 to 9,0 by reacting the substrate, 4-(hydroxymethylphosphinyl)-2-oxo-butyric acid at its initial high concentration with glutamic acid or its salt as added initially in a molar proportion of 0,2 to 3,0 mol per 1 mol of the substrate (OMPB) and in the presence of aspartic acid or its salt as added initially in a molar proportion of 1,0 to 3,0 mol per 1 mol of the substrate (OMPB) and in the presence of cultured cells of such a microorganism which is capable of producing a transaminase having an enzymatic activity to convert the substrate (OMPB) into the desired product, L-2-amino-4-(hydroxymethylphosphinyl)-butyric acid, in the presence of glutamic acid or its salt as the amino-donor and which is also capable of producing a transaminase having a GOT activity to regenerate the by-produced 2-keto-glutaric acid or its salt into glutamic acid or its salt in the presence of aspartic acid or its salt acting as a second amino-donor to give its amino group to the 2-keto-glutaric acid.

2. The process as claimed in claim 1,
in which the substrate compound (OMPB) is dissolved at its initial high concentration of 0,1 to 100 mg/ml in the aqueous reaction medium.

3. The process as claimed in claim 1 or 2,
in which the transamination reaction is effected at a temperature in a range between room temperature and 60 °C.

4. A process as claimed in any of claims 1 to 3, wherein the microorganism used which is capable of producing the transaminase is selected from Streptomyces hygroscopicus SF-1293 FERM BP-130 or ATCC 21705, Streptomyces hygroscopicus NP-50 FERM BP-1368, Streptomyces lividans 66 FERM BP-737, Streptomyces albus IFO 13014 (ATCC 3004), Streptomyces griseus IFO 12875 (ATCC 23345), Streptovericillium cinnamoneum IFO 12852 (ATCC 11874), Streptomyces morookaensis IFO 13416 (ATCC 19166), Nocardia mediterranei ATCC 21271, Nocardiopsis dassonvillei JCM 3237, Streptomyces viridochromogenes IFO 13347 (ATCC 14920), Streptomyces viridochromogenes JCM 4977, Micromonospora carbonaceae NRRL 2972 (ATCC 27114), Escherichia coli ATCC 10798, Pseudomonas aeruginosa ATCC 10145, Pseudomonas cepacia ATCC 17759, Serratia marcescens ATCC 13880, and Mucor spinescens IAM Mu3.

## Patentansprüche

1. Verfahren zur Herstellung von L-2-Amino-4-(hydroxymethylphosphinyl)-buttersäure der Formel (I): 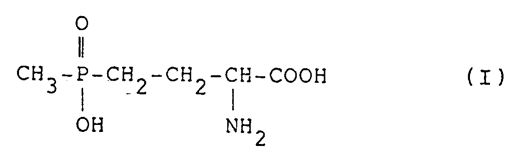 durch Umsetzen von 4-(Hydroxymethylphosphinyl)-2-oxobuttersäure der Formel (II): 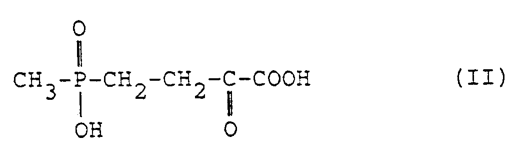 mit Glutaminsäure oder ihrem Salz als Aminodonor für das Substrat, 4-(Hydroxymethylphosphinyl)-2-oxobuttersäure, in Gegenwart einer Transaminase, die fähig ist, die Substratverbindung gemäß Formel (II) in L-Amino-4-(hydroxymethylphosphinyl)-buttersäure gemäß Formel (I) in Gegenwart von Glutaminsäure oder ihrem Salz als Aminodonor umzuwandeln, wobei die Glutaminsäure oder ihr Salz als Aminodonor in 2-Ketoglutarsäure oder ihr Salz als Nebenprodukt unter der Wirkung der Transaminase umgewandelt wird,
dadurch gekennzeichnet,
daß die Transaminierungsreaktion des Substrats 4-(Hydroxymethylphosphinyl)-2-oxobuttersäure gemäß Formel (II) mit Glutaminsäure oder ihrem Salz als dem Aminodonor in einem wäßrigen Reaktionsmedium mit einem alkalischen pH-Wert von 8,0 bis 9,0 wirksam ist durch Umsetzen des Substrats 4-(Hydroxymethylphosphinyl)-2-oxobuttersäure in ihrer ursprünglichen hohen Konzentration mit Glutaminsäure oder ihrem Satz, wie zu Beginn in einem Molverhältnis von 0,2 bis 3,0 mol pro 1 mol des Substrats (OMPB) zugesetzt, und in Gegenwart von Asparaginsäure oder ihrem Satz, wie zu Beginn in einem Molverhältnis von 1,0 bis 3,0 mol pro 1 mol des Substrats (OMPB) zugesetzt, und in Gegenwart von in Kultur gezüchteten Zellen eines Mikroorganismus, der fähig ist, eine Transaminase mit einer enzymatischen Aktivität zu erzeugen, um das Substrat (OMBP) in das gewünschte Produkt L-2-Amino-4-(Hydroxymethylphosphinyl)-buttersäure in Gegenwart von Glutaminsäure oder ihrem Satz als Aminodonor umzuwandeln, und der außerdem fähig ist, eine Transaminase mit einer GOT-Aktivität zu erzeugen, um die als Nebenprodukt erhaltene 2-Ketoglutarsäure oder ihr Salz zu Glutaminsäure oder ihrem Satz in Gegenwart von Asparaginsäure oder ihrem Satz, die als zweiter Aminodonor wirkt, um ihre Aminogruppe an die 2-Ketoglutarsäure abzugeben, zu regenerieren.

2. Verfahren nach Anspruch 1,
wobei die Substratverbindung (OMPB) bei ihrer ursprünglichen hohen Konzentration von 0,1 bis 100 mg/ml in dem wäßrigen Reaktionsmedium gelöst wird.

3. Verfahren nach Anspruch 1 oder 2,
wobei die Transaminierungsreaktion bei einer Temperatur in einem Bereich zwischen Raumtemperatur und 60 °C durchgeführt wird.

4. Verfahren nach einem der Ansprüche 1 bis 3,
wobei der verwendete Mikroorganismus, der fähig ist, die Transaminase zu erzeugen, ausgewählt ist aus Streptomyces hygroscopicus SF-1293 FEPM BP-130 oder ATCC 21705, Streptomyces hygroscopicus NP-50 FERM BP-1368, Streptomyces lividans 66 FERM BP-737, Streptomyces albus IFO 13014 (ATCC 3004), Streptomyces griseus IFO 12875 (ATCC 23345), Streptovericillium cinnamoneum IFO 12852 (ATCC 23345), Streptovericillium cinnamoneum IFO 12852 (ATCC 11874), Streptomyces morookaensis IFO 13416 (ATCC 19166), Nocardia mediterranei ATCC 21271, Nocardionsis dassonvillei JCM 3237, Streptomyces viridochromogenes IFO 13347 (ATCC 14920), Streptomyces viridochromogenes JCM 4977, Micromonospora carbonaceae NRRL 2972 (ATCC 27114), Escherichia coli ATCC 10798; Pseudomonas aeruginosa ATCC 10145, Pseudomonas cepacia ATCC 17759, Serratia marcescens ATCC 13880 und Mucor spinescens IAM Mu3.

## Revendications

1. Procédé pour produire l'acide L-2-amino-4-(hydroxyméthylphosphinyl)-butyrique de la formule (I) : 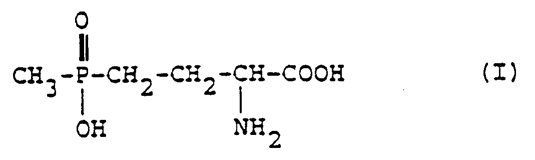 en faisant réagir l'acide 4-(hydroxyméthylphosphinyl)-2-oxo-butyrique de la formule (II) : 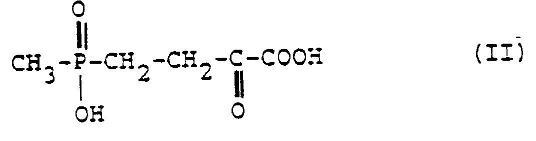 avec l'acide glutamique ou son sel comme donneur de groupes amino au substrat, l'acide 4-(hydroxyméthylphosphinyl)-2-oxo-butyrique, en la présence d'une transaminase susceptible de convertir le composé substrat selon la formule (II) en l'acide L-amino-4-(hydroxyméthylphosphinyl)-butyrique selon la formule (I) en présence de l'acide glutamique ou de son sel comme donneur de groupes amino, et dans lequel l'acide glutamique ou son sel comme donneur de groupes amino est converti en acide 2-céto-glutarique ou son sel comme sous-produit sous l'action de la transaminase,
caractérisé en ce que la réaction de transamination du substrat, l'acide 4-(hydroxyméthylphosphinyl)-2-oxo-butyrique selon la formule (II) avec l'acide glutamique ou son sel comme donneur de groupes amino est effective dans un milieu de réaction aqueux pour une valeur alcaline de pH de 8 à 9 en faisant réagir le substrat, l'acide 4-(hydroxyméthylphosphinyl)-2-oxo-butyrique à sa concentration élevée initiale avec l'acide glutamique ou son sel comme ajouté initialement dans une proportion molaire de 0,2 à 3,0 moles/1 mole du substrat (OMPB) et en la présence d'acide aspartique ou de son sel comme ajouté initialement dans une proportion molaire de 1 à 3 moles/1 mole du substrat (OMPB) et en la présence de cellules cultivées d'un micro-organisme susceptible de produire une transaminase ayant une activité enzymatique pour convertir le substrat (OMPB) en le produit recherché, l'acide L-2-amino-4-(hydroxyméthylphosphinyl)-butyrique, en la présence d'acide glutamique ou de son sel comme donneur de groupes amino, et également susceptible de produire une transaminase ayant une activité GOT pour régénérer l'acide 2-céto-glutarique ou son sel présent comme sous-produit en acide glutamique ou son sel en la présence d'acide aspartique ou de son sel agissant en tant que deuxième donneur de groupes amino pour donner son groupe amino à l'acide 2-céto-glutarique.

2. Procédé selon la revendication 1, dans lequel le composé substrat (OMPB) est dissous à sa concentration élevée initiale de 0,1 à 100 mg/ml dans le milieu de réaction aqueux.

3. Procédé selon la revendication 1 ou la revendication 2, dans lequel la réaction de transamination est effectuée à une température comprise entre la température ambiante et 60°C.

4. Procédé selon l'une des revendications 1 à 3, dans lequel le micro-organisme utilisé et susceptible de produire la transaminase est choisi parmi les suivants : Streptomyces hygroscopicus SF-1293 FERM BP-130 ou ATCC 21705, Streptomyces hygroscopicus NP-50 FERM BP-1368, Streptomyces lividans 66 FERM BP-737, Streptomyces albus IFO 13014 (ATCC 3004), Streptomyces griseus IFO 12875 (ATCC 23345), Streptovericillium cinnamoneum IFO 12852 (ATCC 11874), Streptomyces morookaensis IFO 13416 (ATCC 19166), Nocardia mediterranei ATCC 21271, Nocardiopsis dassonvillei JCM 3237, Streptomyces viridochromogenes IFO 13347 (ATCC 14920), Streptomyces viridochromogenes JCM 4977, Micromonospora carbonaceae NRRL 2972 (ATCC 27114), Escherichia coli ATCC 10798, Pseudomonas aeruginosa ATCC 10145, Pseudomonas cepacia ATCC 17759, Serratia marcescens ATCC 13880 et Mucor spinescens IAM Mu3.
